Europäisches Patentamt

European Patent Office

Office européen des brevets

(19)

(11) Veröffentlichungsnummer : **0 475 036 B1**

(12)

# EUROPÄISCHE PATENTSCHRIFT

(45) Veröffentlichungstag der Patentschrift :
**15.12.93 Patentblatt 93/50**

(51) Int. Cl.$^5$ : **C01G 55/00, B01J 31/40**

(21) Anmeldenummer : **91112477.4**

(22) Anmeldetag : **25.07.91**

(54) **Verfahren zur Wiedergewinnung von Rhodium aus den Rückständen der Destillation von Produkten der Oxosynthese.**

(30) Priorität : **08.08.90 DE 4025074**

(43) Veröffentlichungstag der Anmeldung :
**18.03.92 Patentblatt 92/12**

(45) Bekanntmachung des Hinweises auf die
Patenterteilung :
**15.12.93 Patentblatt 93/50**

(84) Benannte Vertragsstaaten :
**AT BE CH DE DK ES FR GB IT LI LU NL SE**

(56) Entgegenhaltungen :
**EP-A- 0 015 379**

(56) Entgegenhaltungen :
**EP-A- 0 322 661**
**EP-A- 0 348 833**
**EP-A- 0 424 736**
**US-A- 4 390 473**

(73) Patentinhaber : **HOECHST
AKTIENGESELLSCHAFT
D-65926 Frankfurt (DE)**

(72) Erfinder : **Lappe, Peter, Dr. Dipl.-Chem.
Eickenhof 34
W-4220 Dinslaken (DE)**
Erfinder : **Springer, Helmut, Dipl.-Ing.
Borbecker Strasse 19
W-4200 Oberhausen 11 (DE)**

EP 0 475 036 B1

Jouve, 18, rue Saint-Denis, 75001 PARIS

## Beschreibung

Die vorliegende Erfindung betrifft ein verbessertes Verfahren zur Wiedergewinnung von Rhodium aus den Rückständen, die bei der Destillation von Produkten der Oxosynthese erhalten werden.

Die Herstellung von Aldehyden und Alkoholen durch Anlagerung von Kohlenmonoxid und Wasserstoff an olefinische Doppelbindungen (Hydroformylierung) ist bekannt. Die Reaktion wird durch Metalle der 8. Nebengruppe des Periodensystems oder deren Verbindungen katalysiert, die unter den Reaktionsbedingungen Carbonyle oder Hydridocarbonyle bilden. Während früher fast ausschließlich Kobalt und Kobaltverbindungen als Katalysatoren eingesetzt wurden, finden heute in zunehmendem Maße Rhodiumkatalysatoren Anwendung, obgleich Rhodium um ein vielfaches teurer als Kobalt ist. Rhodium gelangt dabei allein oder in Kombination mit Komplexbildnern, z.B. organischen Phosphinen, zum Einsatz. Während die Oxosynthese mit Rhodium als Katalysator Reaktionsdrücke von 25 bis 30 MPa erfordert, genügen bei Einsatz von Rhodium-Komplexverbindungen Drücke von 1 bis 5 MPa.

Für Rhodium-Katalysatoren ergeben sich in vielen Fällen deutliche Vorteile. Sie besitzen höhere Aktivität und Selektivität und ermöglichen überdies einen in vieler Hinsicht problemlosen Betrieb der Produktionsanlage, insbesondere was die Durchführung der Synthese und die Ausbringung der Produkte aus dem Reaktor anbetrifft. Schließlich kann das klassische Oxoverfahren auf Basis von Kobaltkatalysatoren unter Verwendung der vorhandenen Apparateteile in vielen Fällen mit nur geringen Investitionen auf Rhodiumkatalysatoren umgestellt werden.

Erhebliche Schwierigkeiten bereitet jedoch die verlustfreie oder zumindest annähernd verlustfreie Abtrennung und Wiedergewinnung des Rhodiums, unabhängig davon, ob es mit oder ohne zusätzlichem Komplexbildner als Katalysator eingesetzt wird. Nach Beendigung der Umsetzung findet sich das Rhodium als Carbonylverbindung, die gegebenenfalls noch weitere Liganden enthalten kann, gelöst im Hydroformylierungsprodukt.

Zur Aufarbeitung wird das Oxorohprodukt üblicherweise mehrstufig entspannt, indem man den Synthesedruck, der je nach Art des eingesetzten Rhodiumkatalysators etwa 1 bis 30 MPa beträgt, zunächst auf etwa 0,5 bis 2,5 MPa reduziert. Hierbei wird im Rohprodukt gelöstes Synthesegas frei. Anschließend kann man auf Normaldruck entspannen. Die Abtrennung des Rhodiums erfolgt entweder unmittelbar aus dem Rohprodukt oder aus dem Rückstand der Rohproduktdestillation. Der erste Weg wird dann beschritten, wenn in der vorausgegangenen Hydroformylierungsstufe Rhodium ohne zusätzlichen Komplexbildner als Katalyator eingesetzt worden war. Die zweite Variante wird angewandt, wenn der Rhodiumkatalysator außer Kohlenmonoxid noch weitere Liganden, z.B. Phosphine oder Phosphite in komplexer Bindung enthält. Sie kann auch dann genutzt werden, wenn die Hydroformylierung zwar mit Rhodium allein durchgeführt, dem Rohprodukt nach Entspannung aber ein Komplexbildner zur Stabilisierung des Rhodiums zugesetzt worden war. Grundsätzlich ist zu berücksichtigen, daß das Edelmetall im Rohprodukt in einer Konzentration von nur wenigen ppm vorliegt, seine Abtrennung daher sehr sorgfältiges Arbeiten erfordert. Zusätzliche Schwierigkeiten können dadurch entstehen, daß das Rhodium, insbesondere wenn es ohne Ligand eingesetzt wurde, bei der Entspannung teilweise in metallische Form übergeht oder mehrkernige Carbonyle bildet. Es kommt dann zur Ausbildung eines heterogenen Systems, das aus der flüssigen organischen und der festen, Rhodium oder Rhodiumverbindungen enthaltenden Phase besteht.

Die Rückgewinnung von Rhodium aus den Produkten der Oxosynthese, darunter auch den Rückständen von Oxorohprodukten, ist vielfach untersucht worden. Die Arbeiten führten zur Entwicklung zahlreicher Verfahren, von denen einige auch Anwendung im technischen Maßstab gefunden haben.

Gegenstand der US-A- 4 400 547 ist die Hydroformylierung von Olefinen mit 2 bis 20 Kohlenstoffatomen in Gegenwart von unmodifiziertem Rhodium als Katalysator. Nach Beendigung der Reaktion setzt man dem Oxorohprodukt eine komplexbildende Verbindung wie Triphenylphosphin zu und destilliert den Aldehyd ab. Anschließend wird der Destillationsrückstand mit Sauerstoff behandelt, um den Liganden aus der Komplexverbindung wieder abzuspalten und das Rhodium in aktiver Form zurückzugewinnen. Eine Trennung von Rhodium und Destillationsrückstand ist nach dieser Arbeitsweise nicht möglich.

Die Abtrennung von Edelmetallen wie Rhodium aus hochsiedenden Hydroformylierungsrückständen wird auch in der US-A- 3 547 964 beschrieben. Hierzu behandelt man die Rückstände in Gegenwart von Säuren wie Ameisensäure, Salpetersäure oder Schwefelsäure mit Wasserstoffperoxid. Wegen des hohen Preises von Wasserstoffperoxid und seiner problematischen Handhabung sind der technischen Anwendung des Verfahrens jedoch Grenzen gesetzt.

Nach der DE-C- 24 48 005 wird ein Rhodium enthaltender Destillationsrückstand zunächst mit Säuren und Peroxiden behandelt. Darauf zerstört man überschüssige Peroxide durch Erhitzen und setzt die das Katalysatormetall enthaltende wäßrige Lösung in Gegenwart eines wasserlöslichen organischen Lösungsmittels mit Halogenwasserstoffsäure oder Alkalihalogeniden sowie mit tertiären Phosphinen und Kohlenmonoxid oder Kohlenmonoxid abspaltenden Verbindungen um. Diese Arbeitsweise erfordert wiederum die Verwendung von Peroxiden mit den oben geschilderten Nachteilen und den Einsatz halogenbeständiger Werkstoffe.

In der EP-A- 0 015 379 schließlich wird ein Verfahren zum Regenerieren eines Liganden enthaltenden Rhodiumkatalysators beschrieben, der in einer Hydroformylierungsreaktion desaktiviert wurde. Zu diesem Zweck setzt man dem Katalysator Aldehyd in einer solchen Menge hinzu, daß je Mol Rhodium und je Mol Ligand mindestens ein Mol Aldehyd vorliegt. Darauf leitet man Sauerstoff in Form von Luft durch das Gemisch aus Katalysator und Aldehyd, entfernt den festen oxidierten Liganden und stellt das Verhältnis von Ligand zu Rhodium auf den für die Hydroformylierung gewünschten Wert ein. Diese Arbeitsweise erlaubt es zwar, die Aktivität des Katalysators wiederherzustellen, sie ermöglicht aber weder die Wiedergewinnung des Rhodiums noch Rhodium von solchen Verunreinigungen zu befreien, die bei der Einwirkung von Sauerstoff oder Luft nicht oxidiert werden oder keine im organischen Medium schwer- oder unlöslichen und damit abtrennbaren Umwandlungsprodukte ergeben.

Es bestand daher die Aufgabe ein Verfahren zu entwickeln, das die geschilderten Nachteile vermeidet und auf möglichst einfachem Wege eine verlustfreie oder doch annähernd verlustfreie Wiedergewinnung des Edelmetalls sicherstellt.

Erfindungsgemäß wird diese Aufgabe gelöst durch ein Verfahren zur Wiedergewinnung von Rhodium, das in komplexer Bindung mit einer organischen Phosphor(III)-verbindung in den Rückständen der Destillation von Produkten der Oxosynthese enthalten ist durch Behandlung der Rückstände mit Sauerstoff oder einem Sauerstoff enthaltenden Gas. Es ist dadurch gekennzeichnet, daß in einer ersten Reaktionsstufe die Behandlung mit Sauerstoff oder einem Sauerstoff enthaltenden Gas bei 60 bis 120°C drucklos oder unter Druck in Gegenwart einer $C_2$- bis $C_4$-Monocarbonsäure und des Alkalisalzes einer $C_2$- bis $C_4$-Monocarbonsäure erfolgt, darauf das Rhodium aus den Rückständen mit Wasser als wasserlösliche Verbindung extrahiert wird, wäßrige und organische Phase voneinander getrennt werden und die organische Phase in einer zweiten Reaktionsstufe erneut in Gegenwart einer $C_2$- bis $C_4$-Monocarbonsäure und des Alkalisalzes einer $C_2$- bis $C_4$-Monocarbonsäure und unter Zusatz eines Aldehyds bei 60 bis 120°C drucklos oder unter Druck mit Sauerstoff oder einem Sauerstoff enthaltenden Gas behandelt und das in der organischen Phase enthaltene Rhodium durch Extraktion mit Wasser abgetrennt wird.

Die erfindungsgemäße Arbeitsweise erfordert keinen großen apparativen Aufwand oder den Einsatz wertvoller Chemikalien. Dennoch führt sie überraschend zur Wiedergewinnung von mehr als 95% des eingesetzten Rhodiums. Dabei fällt das Metall in einer Form an, die seinen erneuten Einsatz als Katalysator ohne besondere zusätzliche Maßnahmen ermöglicht.

Das neue Verfahren geht von den Rückständen der Hydroformylierung olefinisch ungesättigter Verbindungen aus, die nach dem Abdestillieren der Aldehyde und als Nebenprodukte gebildeter Alkohole im Destillationssumpf zurückbleiben. Sie bestehen im wesentlichen aus höhermolekularen Verbindungen, die aus den Aldehyden durch Aldolkondensation entstanden sind und in einer Folgereaktion unter Bildung ungesättigter Verbindungen auch Wasser abspalten können. Die Art der Verbindungen, die hydroformyliert wurden, ist für die beanspruchte Arbeitsweise ohne Bedeutung. Dementsprechend können sowohl Rückstände eingesetzt werden, die aus der Reaktion von Olefinen mit Kohlenmonoxid und Wasserstoff resultieren, als auch höhermolekulare Produkte, die bei der Umsetzung olefinisch ungesättigter Verbindungen entstehen, die außer der Doppelbindung noch funktionelle Gruppen im Molekül enthalten. Im Vordergrund des neuen Verfahrens steht aber die Wiedergewinnung von Rhodium aus den Rückständen der Hydroformylierung von Olefinen mit 2 bis 12 Kohlenstoffatomen, entsprechend der wirtschaftlichen Bedeutung der aus ihnen hergestellten Aldehyde. Neben den gesättigten und ungesättigten Kondensationsprodukten können die Rückstände auch noch Verbindungen enthalten, die mit den Rhodiumionen unter Komplexbildung reagieren und gegenüber dem Rhodium zumeist im Überschuß vorhanden sind. Zu diesen Verbindungen gehören organische Phosphor(III)-verbindungen insbesondere Phosphine und Phosphite vorzugsweise die Arylverbindungen wie Triphenylphosphin und Triphenylphosphit. Ihre Aufgabe ist es durch Bildung stabiler Komplexverbindungen während der Umsetzung die Selektivität der Reaktion zu verbessern und nach der Umsetzung die Abscheidung metallischen Rhodiums zu verhindern. Im Reaktionsgemisch beträgt das Verhältnis von Ligand und Rhodium bevorzugt 2 bis 150 und insbesondere 5 bis 50 mol/mol. Wegen ihrer geringen Flüchtigkeit liegen beide Komponenten auch im Destillationsrückstand etwa im selben Verhältnis vor, wobei die Rhodiumkonzentration zwischen 30 und 1000 Gew.-ppm, vorzugsweise 100 bis 500 Gew.-ppm beträgt.

Erfindungsgemäß wird der Destillationsrückstand in der ersten Reaktionsstufe mit Sauerstoff behandelt. Das Oxidationsmittel wird in reiner Form eingesetzt oder als Sauerstoff enthaltendes Gasgemisch, insbesondere Luft. Die Sauerstoffmenge kann in weiten Grenzen variiert werden. Sie richtet sich vorzugsweise nach der Konzentration der Phosphor(III)-verbindungen im Rückstand. Es empfiehlt sich je mol Phosphor(III)-verbindung 10 bis 2000, insbesondere 100 bis 1200 mol Sauerstoff anzuwenden.

Entsprechend der Erfindung erfolgt die Umsetzung des Destillationsrückstandes (im folgenden auch als unbehandelter Rückstand bezeichnet) mit Sauerstoff in Gegenwart einer gesättigten, geradkettigen oder verzweigten Monocarbonsäure mit 2 bis 4 Kohlenstoffatomen, Beispiele für geeignete Säuren sind Essigsäure, Propionsäure, n-Buttersäure und i-Buttersäure. Besonders bewährt haben sich Essigsäure und Propionsäure. Sie werden in der handelsüblichen Form und in einer solchen Menge eingesetzt, daß je mol Rhodium etwa 1 bis 150, vorzugsweise 5 bis 50 mol Säure vorhanden sind. Die Säure wird dem Rückstand vor der Umsetzung mit Sauerstoff zugesetzt unabhängig davon, daß sich aufgrund geringer Mengen Aldehyd, die im Rückstand vorhanden sind, im Laufe der Reaktion aus dem Rückstand selbst Säure bilden kann. Die genaue Wirkungsweise der Säure ist nicht bekannt. Verschiedene Beobachtungen sprechen dafür, daß sie eine Startfunktion ausübt, d.h. das Einsetzen der Reaktion maßgebend beeinflußt.

Ein weiteres, ebenfalls sehr wichtiges Merkmal dieser Stufe des erfindungsgemäßen Verfahrens ist die Anwesenheit eines Alkalicarboxylats im Rückstand während Sauerstoff in das Gemisch der hochsiedenden Verbindungen eingeleitet wird. Auch die Art seines Eingreifens in das Reaktionsgeschehen ist nicht eindeutig zu erklären. Es hat sich jedoch gezeigt, daß der Carboxylatzusatz eine deutliche Erhöhung der zurückgewonnenen Rhodiummenge, d.h. eine weitere Verminderung des in der organischen Phase gelöst bleibenden Rhodiums zur Folge hat. Als Alkalicarboxylate werden im Rahmen des neuen Verfahrens Salze gesättigter, geradkettiger oder verzweigter Monocarbonsäuren mit 2 bis 4 Kohlenstoffatomen eingesetzt. Besonders bewährt haben sich die Natrium- und Kaliumsalze der Essigsäure, der Propionsäure und der n- und iso-Buttersäure. Sie werden in einer Menge von 10 bis 250, vorzugsweise 25 bis 100 mol Carboxylat je mol Rhodium angewandt. Geeignet sind die handelsüblichen Salze, die jedoch erst im Verlauf der Oxidation allmählich in Lösung gehen. Vorteilhafter ist es daher, zum Rückstand freie Säure und die äquivalente Menge Alkalihydroxid zu geben, die sofort homogen gelöst und damit voll wirksam werden.

Die Umsetzung des Rückstandes mit Sauerstoff erfolgt bei 60 bis 120, vorzugsweise 80 bis 100°C. Sie kann drucklos oder unter Druck vorgenommen werden, Drücke zwischen 0,2 und 1,0 MPa haben sich besonders bewährt.

Nach einer bevorzugten Ausführungsform der ersten Stufe des erfindungsgemäßen Verfahrens enthalten die Rückstände, auf die Sauerstoff in reiner Form oder im Gemisch mit inerten Gasen einwirkt, Rhodium in einer Konzentration von etwa 100 Gew.-ppm und weniger, vorzugsweise 30 bis 90 Gew.-ppm. Es wurde nämlich gefunden, daß die Rhodiumrestmengen im Rückstand nach der Behandlung dann besonders niedrig sind, wenn die Metallkonzentration in der Ursprungslösung innerhalb der genannten Bereiche liegt. Es empfiehlt sich daher Lösungen, in denen die Rhodiumkonzentration mehr als etwa 100 Gew.-ppm beträgt entsprechend zu verdünnen. Als Verdünnungsmittel geeignet sind insbesondere höher siedende aliphatische oder aromatische Kohlenwasserstoffe oder Kohlenwasserstoffgemische wie Toluol und Xylol oder auch vom Rhodiumkatalysator befreite Destillationsrückstände.

Die Reaktionszeit ist abhängig von der Rhodium- und der Ligandenkonzentration im Destillationsrückstand. Sie wird weiterhin durch die eingesetzte Sauerstoffmenge, durch Reaktionstemperatur und -druck bestimmt. Hohe Konzentrationen der gelösten Substanzen erfordern längere Behandlungszeiten als niedrige Konzentrationen. Ein großes Sauerstoffangebot und erhöhter Druck vermindern die Reaktionszeit ebenso wie eine intensive Durchmischung des Rückstandes mit Sauerstoff. Temperaturen im unteren und im oberen Bereich des beanspruchten Intervalls sind etwas weniger effektiv als im mittleren Bereich.

Nach Abschluß der Behandlung mit Sauerstoff wird die organische Phase mit Wasser extrahiert. Man arbeitet bei Temperaturen zwischen 20 und 80°C, vorzugsweise bei 30 bis 70°C in einer, zweckmäßiger in mehreren Stufen. Die eingesetzte Wassermenge richtet sich nach dem Verteilungsgleichgewicht der zu extrahierenden Substanz zwischen organischer und wäßriger Phase und der angestrebten Rhodiumkonzentration in der wäßrigen Phase.

Sobald die Extraktion beendet ist, trennt man die Phasen voneinander und läßt erfindungsgemäß in der zweiten Reaktionsstufe erneut Sauerstoff in reiner Form oder in Mischung mit inerten Gasen auf die organische Phase einwirken. Hierbei kann die restliches Rhodium enthaltende organische Lösung unmittelbar, d.h. so wie sie nach der Extraktion anfällt, verwendet werden. Eine vorherige Trocknung ist ebensowenig erforderlich, wie eine Entfernung des Lösungsmittels, das dem Rückstand gegebenenfalls zugesetzt wurde. Die Behandlung des Destillationsrückstandes in der zweiten Reaktionsstufe unterscheidet sich von der Behandlung in der ersten Reaktionsstufe vor allem dadurch, daß man der Rhodium enthaltenden Lösung vor der Umsetzung mit Sauerstoff einen Aldehyd zusetzt. Bewährt haben sich aliphatische Aldehyde mit 2 bis 4 Kohlenstoffatomen im Molekül, bevorzugt werden Acetaldehyd und Propionaldehyd. Die dem Reaktionsgemisch zuzusetzende Aldehydmenge richtet sich nach der Menge des Rückstandes. Es hat sich bewährt, 0,01 bis 2,0 kg Aldehyd je kg Rückstand zu verwenden, bevorzugt wird ein Verhältnis von 0,05 bis 1,0 kg Aldehyd je kg Rückstand. Selbstverständlich ist es nicht erforderlich, die Aldehyde in Form der reinen Verbindungen anzuwenden. Mit gleich gutem Erfolg können auch Aldehydgemische benutzt werden, z.B. Gemische aus strukturisomeren Aldehyden oder aus Aldehyden unterschiedlicher Molekülgröße.

Neben einem Aldehyd werden dem Reaktionsgemisch, wie in der ersten Stufe, eine gesättigte, geradkettige oder verzweigte Monocarbonsäure mit 2 bis 4 Kohlenstoffatomen und das Alkalisalz einer solchen Carbonsäure zugegeben. Es ist nicht erforderlich, daß Carbonsäure und Salz in den beiden Reaktionsstufen gleich sind, vielmehr können in den beiden Stufen auch unterschiedliche Säuren und/oder Salze verwendet werden. Vorteilhaft ist es jedoch wiederum, das Carboxylat in der organischen Lösung aus Säure und der äquivalenten Menge Alkalihydroxid unmittelbar herzustellen, um sicherzustellen, daß es sofort und homogen gelöst wird.

Grundsätzlich kann man in der zweiten Reaktionsstufe mit demselben Rhodium/Carbonsäure- bzw. Rhodium/Carboxylat-Verhältnis arbeiten wie in der ersten Stufe, d.h. je mol Rhodium 1 bis 150, vorzugsweise 5 bis 50 mol Säure und 10 bis 250, vorzugsweise 25 bis 100 mol Carboxylat anwenden. Es hat sich jedoch als zweckmäßig erwiesen, in der zweiten Stufe mit derselben, absoluten Menge Carbonsäure bzw. Carboxylat zu arbeiten. Berücksichtigt man, daß der Destillationsrückstand nach der ersten Reaktionsstufe nur noch etwa ein Zehntel der ursprünglich vorhandenen Rhodiummenge enthält, dann resultiert in der zweiten Stufe ein zehnfach höherer Carbonsäure- bzw. Carboxylatüberschuß als in der ersten Stufe. Je mol Rhodium sind also 10 bis 1500 mol Säure und 100 bis 2500 mol Salz anzuwenden.

Durch die gegebenenfalls noch ein Lösungsmittel enthaltende Lösung leitet man reinen Sauerstoff oder Sauerstoff verdünnt mit inerten Gasen in großem Überschuß zu dem als gelöste Verbindung vorliegenden Rhodium. Die Bedingungen, unter denen die Sauerstoffbehandlung erfolgt, sind die gleichen, wie in der ersten Stufe. Die Umsetzung erfolgt bei 60 bis 120, vorzugsweise 80 bis 100°C, drucklos oder unter Druck. Besonders bewährt haben sich Drücke zwischen 0,2 und 1,0 MPa.

Die Reaktionszeit hängt, wie in der ersten Stufe, außer von der Rhodiumkonzentration, wesentlich von Druck und Temperatur und vom Sauerstoffangebot, d.h. der absoluten Sauerstoffmenge und der Verteilung des Sauerstoffs in der flüssigen Phase ab. Die für die erste Stufe beschriebenen Auswirkungen von Änderungen der genannten Parameter gelten auch für die zweite Stufe des erfindungsgemäßen Verfahrens.

Nachdem die Behandlung mit Sauerstoff abgeschlossen ist, wird der Rückstand erneut mit Wasser extrahiert. Auch dieser Teilschritt der zweiten Reaktionsstufe wird, wie für die erste Stufe beschrieben, durchgeführt. Bevorzugt extrahiert man mit der in der ersten Reaktionsstufe erhaltenen Rhodiumsalzlösung. Es ist darüber hinaus möglich, diese Lösung wiederholt zur Extraktion zu verwenden, um eine Anreicherung des Metalls in der Lösung zu erzielen. Die wäßrige Lösung kann unmittelbar, d.h. ohne zusätzliche Reinigungsoperationen, zur Katalysatorherstellung verwendet werden.

Die Umsetzung des Destillationsrückstandes kann in konventionellen Apparaturen kontinuierlich oder diskontinuierlich durchgeführt werden. Der Sauerstoff oder das Sauerstoff enthaltende Gas wird über Verteilungsvorrichtungen in den Reaktor geleitet, die gleichmäßige Vermischung von flüssiger und gasförmiger Phase gegebenenfalls durch Rühren unterstützt.

Das neue Verfahren ermöglicht es, mehr als 95 % des in den Destillationsrückständen von Produkten der Hydroformylierung enthaltenen Rhodiums zurückzugewinnen. Besondere Bedeutung erhält die erfindungsgemäße Arbeitsweise dadurch, daß sie ganz allgemein auf Produkte solcher Reaktionen angewendet werden kann, die unter dem katalytischen Einfluß homogen gelöster Rhodiumverbindungen ablaufen. Es war nicht zu erwarten, daß die Kombination zweier unterschiedlicher Reaktionsschritte, von denen jeder für sich geeignet ist, Rhodiumverbindungen, wenn auch unvollständig, aus organischen Lösungen abzutrennen, zu besseren Ergebnissen führt, als die wiederholte Anwendung jedes der beiden Schritte.

Die folgenden Beispiele erläutern die Erfindung. Es ist aber selbstverständlich nicht beabsichtigt, sie auf diese Ausführungsformen zu beschränken.

In Tabelle 1 sind die Einsatzstoffe durch ihre wesentlichen Kennzahlen charakterisiert.

Die Beispiele 1, 4, 5 und 8 geben das erfindungsgemäße zweistufige Verfahren wieder. Es wird in den Beispielen 2 und 7 mit einer Arbeitsweise verglichen, die der zweiten Stufe des neuen Prozesses entspricht und in den Beispielen 3 und 6 mit einem Verfahren, in dem die erste Stufe des Prozesses der Erfindung zweimal hintereinander angewendet wird.

## Tabelle 1

| Einsatzstoffe | Stoff A | Stoff B |
|---|---|---|
| Rh-Gehalt (ppm) | 196 | 436 |
| P(III) (mmol/kg) | 30 | 33 |
| P ges. (mmol/kg) | 31,7 | 54,8 |

Stoff A: Destillationsrückstand aus der Hydroformylierung
von Ethylen bei 130 bis 180°C und 20 bis 30 MPa

Stoff B: Destillationsrückstand aus der Hydroformylierung
von Propylen bei 130 bis 180°C und 20 bis 30 MPa

Beispiel 1

In einem mit Mantelbeheizung versehenen 1 l-Glasautoklaven werden 214,3 g Destillationsrückstand A (Rh-Gehalt: 42 mg), 385,7 g Xylol, 8,2 g 30 %ige Natronlauge sowie 5,1 g 99,5 %ige Propionsäure vorgelegt und unter Rühren innerhalb von 15 Minuten auf 80°C erwärmt. Anschließend leitet man durch ein Tauchrohr über einen Zeitraum von 3 h und unter einem Druck von 0,35 MPa je Stunde 45 l Luft ein. Die Umsetzung erfolgt bei konstantem Innendruck von 0,35 MPa und konstanter Temperatur von 80°C. Das Abgas wird über ein Nadelventil im Deckel des Autoklaven entspannt und in einen mit Kühler versehenen Kolben geleitet.

Nach Beendigung der Reaktion kühlt man den Autoklaveninhalt in etwa 15 Minuten auf 60°C und stellt die Luftzufuhr ein. Anschließend wird entspannt, das Reaktionsgemisch mit 200,0 g Wasser versetzt und weitere 15 Minuten bei 50 bis 60°C gerührt. Man entnimmt das Produkt dem Reaktor, trennt die Phasen und extrahiert die organische Phase weitere zweimal mit je 200,0 g Wasser. Nach der Behandlung enthält die organische Phase noch 2,8 mg Rh entsprechend 6,6 % des im Einsatzstoff enthaltenen Rh.

Die organische Phase (563,7 g) wird nach Zusatz von 50 g Propionaldehyd, 8,2 g 30 %iger Natronlauge und 5,1 g 99,5 %iger Propionsäure entsprechend der ersten Stufe, mit Luft behandelt. Nach dreimaliger Extraktion mit Wasser wie in Stufe 1 enthält die organische Phase noch 0,3 mg Rh, entsprechend 0,7 % des im Einsatzstoff enthaltenen Rh.

Beispiel 2 (Vergleich)

Analog Beispiel 1 werden 214,3 g Destillationsrückstand A, 335,7 g Xylol, 8,2 g 30 %ige Natronlauge, 5,1 g 99,5 %ige Propionsäure sowie 50 g Propionaldehyd vorgelegt und unter den in Beispiel 1 genannten Bedingungen oxidativ umgesetzt. Nach der Behandlung enthält die organische Phase noch 2,5 mg Rh, entsprechend 6,0 % des im Einsatzstoff enthaltenen Metalls.

Beispiel 3 (Vergleich)

Analog Beispiel 1 werden die entsprechenden Mengen Destillationsrückstand, Xylol, Natronlauge und Propionsäure eingesetzt. Nach der zweistufigen Aufarbeitung - ohne Zusatz von Propionaldehyd in der zweiten Stufe - und Extraktion wie in Beispiel 1, enthält die organische Phase noch 2,7 mg Rh entsprechend 6,4 % des im Einsatzstoff enthaltenen Metalls.

Beispiel 4

In einem mit Mantelbeheizung versehenen 1 l-Glasautoklaven werden 260,7 g Destillationsrückstand A, 339,3 g Xylol, 8,2 g 30 %ige Natronlauge sowie 5,1 g 99,5 %ige Propionsäure vorgelegt und unter Rühren in-

6

nerhalb von 15 Minuten auf 80°C erwärmt. Anschließend leitet man durch ein Tauchrohr über einen Zeitraum von 3 h und unter einem Druck von 0,33 MPa je Stunde 45 l Luft ein. Die Umsetzung erfolgt bei konstantem Innendruck von 0,35 MPa und konstanter Temperatur von 80°C. Das Abgas wird über ein Nadelventil im Deckel des Autoklaven entspannt und in einen mit Kühler versehenen Kolben geleitet.

Nach Beendigung der Reaktion kühlt man den Autoklaveninhalt in etwa 15 Minuten auf 60°C und stellt die Luftzufuhr ein. Anschließend wird entspannt, das Reaktionsgemisch mit 200,0 g Wasser versetzt und weitere 15 Minuten bei 50 bis 60°C gerührt. Man entnimmt das Produkt dem Reaktor, trennt die Phasen und extrahiert die organische Phase weitere zweimal mit je 200,0 g Wasser. Nach der Behandlung enthält die organische Phase noch 4,0 mg Rh entsprechend 7,8 % des im Einsatzstoff enthaltenen Metalls.

Die organische Phase (543,7 g) wird nach Zusatz von 50 g Propionaldehyd, 8,2 g 30 %ige Natronlauge und 5,1 g 99,5 %ige Propionsäure entsprechend der ersten Stufe mit Luft behandelt. Nach dreimaliger Extraktion mit Wasser wie in Stufe 1 enthält die organische Phase noch 1,2 mg Rh, entsprechend 2,3 % des im Einsatzstoff enthaltenen Metalls.

## Beispiel 5

Analog Beispiel 1 werden 96,3 g Destillationsrückstand B, 503,7 g Xylol, 8,2 g 30 %ige Natronlauge und 5,1 g 99,5 %ige Propionsäure vorgelegt und bei einer Temperatur von 80°C, einem Druck von 0,35 MPa und einer Luftmenge von 47,7 l/h über einen Zeitraum von 6 Stunden umgesetzt. Nach Beendigung der Reaktion wird die Lösung wie in Beispiel 1 aufgearbeitet, die organische Phase enthält 9,5 mg Rh entsprechend 22,6 % des im Einsatzstoff enthaltenen Metalls.

Die organische Phase (573,7 g) wird nach Zuatz von 50 g Propionaldehyd, 8,2 g 30 %ige Natronlauge und 5,1 g 99,5 %ige Propionsäure entsprechend der ersten Stufe (Reaktionszeit: 4 Stunden) mit Luft behandelt. Nach dreimaliger Extraktion mit Wasser wie in der ersten Stufe enthält die organische Phase noch 2,0 mg Rh, entsprechend 4,8 % des im Einsatzstoff enthaltenen Metalls.

## Beispiel 6 (Vergleich)

Analog Beispiel 1 werden 96,3 g Destillationsrückstand B, 503,7 g Xylol, 8,2 g 30 %ige Natronlauge und 5,1 g 99,5 %ige Propionsäure vorgelegt und bei einer Temperatur von 80°C, einem Druck von 0,35 MPa und einer Luftmenge von 47,7 l/h über einen Zeitraum von 6 Stunden umgesetzt.

Nach Beendigung der Reaktion wird die Lösung wie in Beispiel 1 aufgearbeitet, die organische Phase enthält 9,5 mg Rh entsprechend 22,6 % des im Einsatzstoff enthaltenen Metalls.

Die organische Phase (537,7 g) wird nach Zusatz von 8,2 g 30 %iger Natronlauge und 5,1 g 99,5 %iger Propionsäure entsprechend der ersten Stufe, also ohne Zugabe von Propionaldehyd, aufgearbeitet (Reaktionszeit: 4 Stunden). Nach dreimaliger Extraktion mit Wasser enthält die organische Phase noch 7,3 mg Rh, entsprechend 17,4 % des im Einsatzstoff enthaltenen Metalls.

## Beispiel 7 (Vergleich)

Analog Beispiel 3 werden 96,3 g Destillationsrückstand B, 453,7 g Xylol, 8,2 g 30 %ige Natronlauge, 5,1 g 99,5 %ige Propionsäure sowie 50,0 g Propionaldehyd vorgelegt und unter den in Beispiel 5 genannten Bedingungen mit Luft behandelt. Nach Aufarbeitung durch Extraktion wie in Beispiel 1 enthält die organische Phase noch 4,7 mg Rh, entsprechend 11,2 % des im Einsatzstoff enthaltenen Metalls.

## Beispiel 8

Analog Beispiel 1 werden 70,0 g Destillationsrückstand B, 530,0 g Xylol, 8,2 g 30 %ige Natronlauge und 5,1 g 99,5 %ige Propionsäure vorgelegt und bei einer Temperatur von 80°C, einem Druck von 0,35 MPa und einer Luftmenge von 47,7 l/h über einen Zeitraum von 6 Stunden umgesetzt. Nach Beendigung der Reaktion wird entsprechend Beispiel 1 aufgearbeitet, die organische Phase enthält 6,3 mg Rh entsprechend 20,6 % des im Einsatzstoff enthaltenen Metalls.

Die organische Phase (581,2 g) wird nach Zusatz von 50 g Propionaldehyd, 8,2 g 30 %ige Natronlauge und 5,1 g 99,5 %ige Propionsäure entsprechend der ersten Stufe aufgearbeitet (Reaktionszeit: 4 Stunden). Nach dreimaliger Extraktion mit Wasser enthält die organische Phase noch 1,2 mg Rh, entsprechend 3,9 % des im Einsatzstoff enthaltenen Metalls.

EP 0 475 036 B1

**Patentansprüche**

1. Verfahren zur Wiedergewinnung von Rhodium, das in komplexer Bindung mit einer organischen Phosphor(III)-verbindung in den Rückständen der Destillation von Produkten der Oxosynthese enthalten ist durch Behandlung der Rückstände mit Sauerstoff oder einem Sauerstoff enthaltenden Gas, dadurch gekennzeichnet, daß in einer ersten Reaktionsstufe die Behandlung mit Sauerstoff oder einem Sauerstoff enthaltenden Gas bei 60 bis 120°C drucklos oder unter Druck in Gegenwart einer $C_2$- bis $C_4$-Monocarbonsäure und des Alkalisalzes einer $C_2$- bis $C_4$-Monocarbonsäure erfolgt, darauf das Rhodium aus den Rückständen mit Wasser als wasserlösliche Verbindung extrahiert wird, wäßrige und organische Phase voneinander getrennt werden und die organische Phase in einer zweiten Reaktionsstufe erneut in Gegenwart einer $C_2$- bis $C_4$-Monocarbonsäure und des Alkalisalzes einer $C_2$- bis $C_4$-Monocarbonsäure und unter Zusatz eines Aldehyds bei 60 bis 120°C drucklos oder unter Druck mit Sauerstoff oder einem Sauerstoff enthaltenden Gas behandelt und das in der organischen Phase enthaltene Rhodium durch Extraktion mit Wasser abgetrennt wird.

2. Verfahren nach Anspruch 1, dadurch gekennzeichnet, daß als Sauerstoff enthaltendes Gas in jeder der beiden Reaktionsstufen Luft verwendet wird.

3. Verfahren nach Anspruch 1 oder 2, dadurch gekennzeichnet, daß in der ersten Reaktionsstufe je mol Phosphor(III)-verbindung 10 bis 2000, insbesondere 100 bis 1200 mol Sauerstoff angewendet werden.

4. Verfahren nach einem oder mehreren der Ansprüche 1 bis 3, dadurch gekennzeichnet, daß dem Rückstand in jeder der beiden Reaktionsstufen jeweils etwa 1 bis 150, vorzugsweise 5 bis 50 mol Monocarbonsäure je mol im unbehandelten Rückstand enthaltenen Rhodium zugesetzt wird.

5. Verfahren nach einem oder mehreren der Ansprüche 1 bis 4, dadurch gekennzeichnet, daß als $C_2$- bis $C_4$-Monocarbonsäure in beiden Reaktionsstufen Essigsäure oder Propionsäure zugesetzt wird.

6. Verfahren nach einem oder mehreren der Ansprüche 1 bis 5, dadurch gekennzeichnet, daß dem Rückstand in jeder der beiden Reaktionsstufen 10 bis 250, vorzugsweise 25 bis 100 mol Alkalisalz einer $C_2$- bis $C_4$-Monocarbonsäure je mol im unbehandelten Rückstand enthaltenen Rhodium zugesetzt wird.

7. Verfahren nach einem oder mehreren der Ansprüche 1 bis 6, dadurch gekennzeichnet, daß als Alkalisalz einer $C_2$- bis $C_4$-Monocarbonsäure das Natrium- oder Kaliumsalz der Essigsäure, der Propionsäure, der n- oder der i-Buttersäure eingesetzt wird.

8. Verfahren nach einem oder mehreren der Ansprüche 1 bis 7, dadurch gekennzeichnet, daß die Behandlung des Rückstandes mit Sauerstoff oder einem Sauerstoff enthaltenden Gas in jeder der beiden Reaktionsstufen jeweils bei 60 bis 120, vorzugsweise 80 bis 100°C und bei Drücken von 0,2 bis 1,0 MPa erfolgt.

9. Verfahren nach einem oder mehreren der Ansprüche 1 bis 8, dadurch gekennzeichnet, daß die Rhodiumkonzentration im unbehandelten Rückstand, gegebenenfalls nach Zusatz eines Verdünnungsmittels, etwa 100 Gew.-ppm und weniger, vzw. 30 bis 90 Gew.-ppm beträgt.

10. Verfahren nach einem oder mehreren der Ansprüche 1 bis 9, dadurch gekennzeichnet, daß dem Rückstand in der zweiten Reaktionsstufe Acetaldehyd oder Propionaldehyd zugesetzt wird.

11. Verfahren nach einem oder mehreren der Ansprüche 1 bis 10, dadurch gekennzeichnet, daß in der zweiten Reaktionsstufe je kg Rückstand 0,01 bis 2 kg, vzw. 0,05 bis 1 kg Aldehyd zugesetzt werden.

12. Verfahren nach einem oder mehreren der Ansprüche 1 bis 11, dadurch gekennzeichnet, daß man das in der organischen Phase enthaltene Rhodium in jeder der beiden Reaktionsstufen mit Wasser bei 20 bis 80, vorzugsweise 30 bis 70°C, in einer oder in mehreren Stufen extrahiert.

**Claims**

1. A process for the recovery of rhodium which is present in a complex combination with an organic phosphorus(III) compound in the residues from the distillation of products of the oxo process, by treating the

8

residues with oxygen or a gas containing oxygen, which comprises treatment with oxygen or a gas containing oxygen in an initial reaction stage at 60 to 120°C and under normal pressure or under pressure in the presence of a $C_2$- to $C_4$-monocarboxylic acid and the alkali metal salt of a $C_2$- to $C_4$-monocarboxylic acid, then extracting the rhodium in the form of a water-soluble compound from the residues by means of water, separating the aqueous and organic phases from one another and treating the organic phase again with oxygen or a gas containing oxygen under normal pressure or under pressure and at 60 to 120°C in a second reaction stage in the presence of a $C_2$- to $C_4$-monocarboxylic acid and the alkali metal salt of a $C_2$- to $C_4$-monocarboxylic acid and with the addition of an aldehyde, and removing the rhodium present in the organic phase by extraction with water.

2. The process as claimed in claim 1, wherein air is used in each of the two reaction stages as the gas containing oxygen.

3. The process as claimed in claim 1 or 2, wherein 10 to 2000, in particular 100 to 1200, mol of oxygen are used per mol of phosphorus(III) compound in the first reaction stage.

4. The process as claimed in one or more of claims 1 to 3, wherein in each case about 1 to 150, preferably 5 to 50, mol of monocarboxylic acid per mol of rhodium present in the untreated residue are added to the residue in each of the two reaction stages.

5. The process as claimed in one or more of claims 1 to 4, wherein acetic acid or propionic acid is added in both reaction stages as the $C_2$- to $C_4$-monocarboxylic acid.

6. The process as claimed in one or more of claims 1 to 5, wherein 10 to 250, preferably 25 to 100, mol of alkali metal salt of a $C_2$- to $C_4$-monocarboxylic acid per mol of rhodium present in the untreated residue are added to the residue in each of the two reaction stages.

7. The process as claimed in one or more of claims 1 to 6, wherein the sodium or potassium salt of acetic acid, propionic acid, n-butyric acid or isobutyric acid is employed as the alkali metal salt of a $C_2$- to $C_4$-monocarboxylic acid.

8. The process as claimed in one or more of claims 1 to 7, wherein the treatment of the residue with oxygen or a gas containing oxygen is in each case carried out at 60 to 120°C, preferably 80 to 100°C, and under pressures of 0.2 to 1.0 mPa in each of the two reaction stages.

9. The process as claimed in one or more of claims 1 to 8, wherein the rhodium concentration in the untreated residue, if appropriate after the addition of a diluent, is about 100 ppm by weight or less, preferably 30 to 90 ppm by weight.

10. The process as claimed in one or more of claims 1 to 9, wherein acetaldehyde or propionaldehyde is added to the residue in the second reaction stage.

11. The process as claimed in one or more of claims 1 to 10, wherein 0.01 to 2 kg, preferably 0.05 to 1 kg, of aldehyde per kg of residue is added in the second reaction stage.

12. The process as claimed in one or more of claims 1 to 11, wherein the rhodium present in the organic phase is extracted, in each of the two reaction stages, with water at 20 to 80°C, preferably 30 to 70°C, in one or more stages.

**Revendications**

1. Procédé pour récupérer le rhodium contenu en liaison complexe avec un composé organique du phosphore-III dans les résidus de la distillation de produits de l'oxosynthèse par traitement de ces résidus à l'aide d'oxygène ou d'un gaz contenant de l'oxygène, caractérisé en ce que, dans un premier stade de réaction, on traite par l'oxygène ou un gaz contenant de l'oxygène à des températures de 60 à 120°C, à pression normale ou sous pression, en présence d'un acide monocarboxylique en $C_2$-$C_4$ et du sel alcalin d'un acide monocarboxylique en $C_2$-$C_4$, on extrait le rhodium des résidus par l'eau à l'état de composé soluble dans l'eau, on sépare la phase aqueuse et la phase organique et, dans un deuxième stade de réaction, on traite à nouveau la phase organique en présence d'un acide monocarboxylique en $C_2$-$C_4$ et du sel alcalin d'un acide monocarboxylique en $C_2$-$C_4$ et avec adjonction d'un aldéhyde, à des températures de 60 à 120°C, à pression normale ou sous pression, par l'oxygène ou un gaz contenant de l'oxygène, et on sépare le rhodium contenu dans la phase organique par extraction à l'eau.

2. Procédé selon la revendication 1, caractérisé en ce que, dans chacun des deux stades de réaction, on utilise l'air en tant que gaz contenant de l'oxygène.

3. Procédé selon la revendication 1 ou 2, caractérisé en ce que, au premier stade de réaction, on utilise de 10 à 2 000, plus spécialement de 100 à 1 200, mol d'oxygène par mole de composé du phosphore-III.

4. Procédé selon une ou plusieurs des revendications 1 à 3, caractérisé en ce que, dans chacun des deux stades de réaction, on ajoute d'environ 1 à 150, de préférence de 5 à 50, mol d'acide monocarboxylique par mole de rhodium contenu dans le résidu non traité.

5. Procédé selon une ou plusieurs des revendications 1 à 4, caractérisé en ce que, dans les deux stades de réaction, on ajoute en tant qu'acide monocarboxylique en $C_2$-$C_4$ l'acide acétique ou l'acide propionique.

6. Procédé selon une ou plusieurs des revendications 1 à 5, caractérisé en ce que, dans chacun des deux stades de réaction, on ajoute au résidu de 10 à 250, de préférence de 25 à 100, mol de sel alcalin d'un acide monocarboxylique en $C_2$-$C_4$ par mole de rhodium contenu dans le résidu non traité.

7. Procédé selon une ou plusieurs des revendications 1 à 6, caractérisé en ce que l'on utilise en tant que sel alcalin d'un acide monocarboxylique en $C_2$-$C_4$ le sel de sodium ou de potassium de l'acide acétique, de l'acide propionique, de l'acide n- ou iso-butyrique.

8. Procédé selon une ou plusieurs des revendications 1 à 7, caractérisé en ce que, dans chacun des deux stades de réaction, le traitement du résidu par l'oxygène ou un gaz contenant de l'oxygène est réalisé à des températures de 60 à 120, de préférence de 80 à 100°C et à des pressions de 0,2 à 1,0 MPa.

9. Procédé selon une ou plusieurs des revendications 1 à 8, caractérisé en ce que la concentration en rhodium du résidu non traité, éventuellement après adjonction d'un diluant, est d'environ 100 ppm en poids ou moins, ou de 30 à 90 ppm en poids.

10. Procédé selon une ou plusieurs des revendications 1 à 9, caractérisé en ce que, au deuxième stade de réaction, on ajoute au résidu de l'acétaldéhyde ou du propionaldéhyde.

11. Procédé selon une ou plusieurs des revendications 1 à 10, caractérisé en ce que, au deuxième stade de réaction, on ajoute de 0,01 à 2 kg ou de 0,05 à 1 kg d'aldéhyde par kg du résidu.

12. Procédé selon une ou plusieurs des revendications 1 à 11, caractérisé en ce que, dans chacun des deux stades de réaction, on extrait le rhodium contenu dans la phase organique par l'eau à des températures de 20 à 80, de préférence de 30 à 70°C, en un ou plusieurs stades.